# EUROPEAN PATENT APPLICATION

(11) **EP 2 515 150 A1**
(43) Date of publication of application: **24.10.2012**
(21) Application number: 09852247.7
(22) Date of filing: 14.12.2009
(51) Int. Cl.: G02B 6/32, A61B 1/00, A61B 10/00, G01N 21/17, G02B 6/02

(54) **LATERAL EMISSION APPARATUS AND MANUFACTURING METHOD THEREOF**

(71) Applicant: Toyo Glass Co., Ltd., Tokyo 141-8640 (JP)
(72) Inventor: SUZUKI Taro, Kawasaki-shi Kanagawa 210-0863 (JP); IEDA Sadayoshi, Kawasaki-shi Kanagawa 210-0863 (JP); MARUYAMA Naofumi, Kawasaki-shi Kanagawa 210-0863 (JP)
(74) Representative: Brown, David Leslie
(86) International application number: PCT/JP2009/070804
(87) International publication number: WO 2011/074051

(57) **Abstract**

A lateral light emitting device is developed. The lateral light emitting device is free from variations and degradation in beam quality and reduction in reliability caused by adhesive, can be easily produced, and has a small outer diameter in order to be usable for a thin blood vessel and the like. The above described problem is solved by the lateral light emitting device, which includes an optical fiber, a rod lens fused to an end of the optical fiber, and a prism having a polygonal section fused to a distal end surface of the rod lens.

## Description

### Technical Field

The present invention relates to a lateral light emitting device that emits light, which has propagated in an optical fiber, in a lateral direction at an angle related to the optical axis of an optical fiber, and, in particular, is suitable for use as an optical probe for OCT (Optical Coherence Tomography). The present invention also relates to a method of producing the lateral light emitting device.

### Background Art

OCT is a method of creating an optical coherence tomographic image that allows a precise tomographic image of the inside of a subject body to be obtained. OCT uses light reflected back from parts of the subject body in response to low-coherence light laterally emitted from the distal end of an optical probe inserted into an organ of the patient such as a blood vessel or the bowels. A basic technology of the OCT is disclosed in Japanese Examined Patent Application Publication No. 6-35946 (Patent Literature 1), and specific structures of the optical probes are disclosed in Japanese Unexamined Patent Application Publication No. 11-56786 (Patent Literature 2), Japanese Unexamined Patent Application Publication No. 2008-200283 (Patent Literature 3), and so forth.

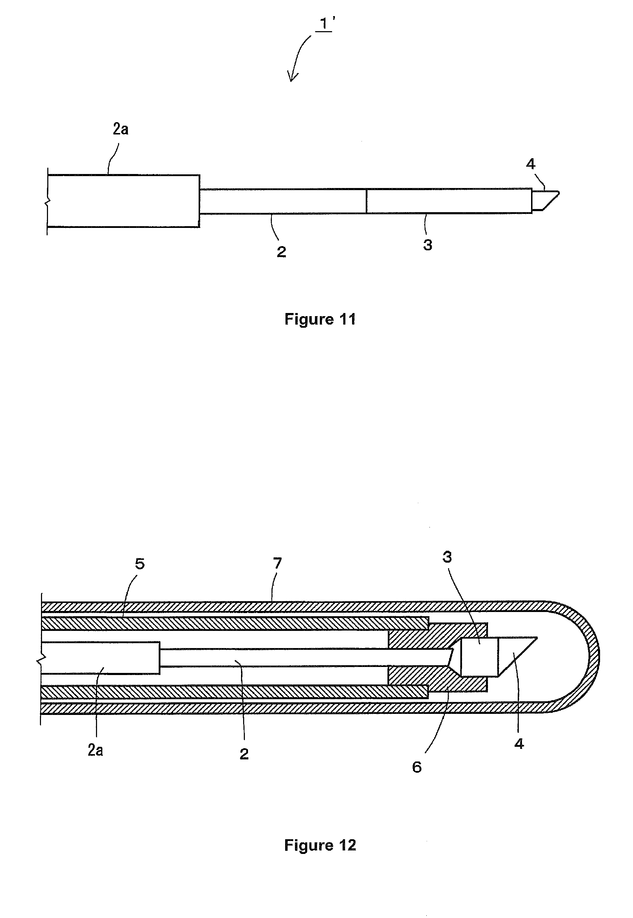

Fig. 12 is a sectional explanatory view of a related-art lateral light emitting device (optical probe) described in Patent Literatures 2 and 3.
A distal end holding portion 6 is mounted at a distal end of a cylindrical shaft 5, a distal end of an optical fiber 2 that is inserted through the shaft 5 is engaged with a proximal end side of the distal end holding portion 6, and a rod lens 3, to which a prism 4 is bonded, is engaged with a distal end side of the distal end holding portion 6. The entirety of the lateral light emitting device is covered with a transparent sheath 7 in order to prevent the subject body from being damaged and prevent the prism 4 and the rod lens 3 from becoming completely detached and being left inside the subject body.

### Citation List

### Patent Literature

Patent Literature 1: Japanese Examined Patent Application Publication No. 6-35946
Patent Literature 2: Japanese Unexamined Patent Application Publication No. 11-56786
Patent Literature 3: Japanese Unexamined Patent Application Publication No. 2008-200283

### Summary of Invention

### Technical Problem

In the related-art lateral light emitting device, the rod lens and the prism are bonded to each other with adhesive, and accordingly, there is an adhesive layer in an optical path. Thus, there are problems such as variations in quality of a beam, and degradation of quality of a beam due to a gap between the rod lens and prism caused by, for some reason, detachment of the adhesion that bonds the rod lens and the prism together. In the worst case, a problem of the lens becoming completely detached from the prism occurs. In order to solve the above-described problems, a method may be adopted in which the distal end of the rod lens is directly ground so as to form an inclined surface portion that will reflect light. However, when light is emitted from the inside of the rod lens to the outside of the rod lens through a side surface of the rod lens, this causes a problem in that emitted beams are affected by the curvature of the side surface and has an elliptic shape, thereby enlarging an illumination area, and accordingly, preventing sufficient spatial resolution from being obtained.
Since the optical fiber and the rod lens need to be engaged with the distal end holding portion 6 at the distal end of the shaft 5 with high precision, assembly is complex, thereby reducing efficiency in production.
Since the shaft 5 and the sheath 7 are essential components, the outer diameter needs to be large, thereby causing a problem in that insertion into a very thin blood vessel or the like is impossible.

An object of the present invention is to solve the problems of the related-art lateral light emitting device as above and to develop a lateral light emitting device that is free from variations and degradation in beam quality and reduction in reliability caused by adhesive, can be easily produced, and has a small outer diameter in order to be usable even for thin blood vessels and the like.

### Solution to Problem

### [Claim 1]

According to the present invention, a lateral light emitting device includes an optical fiber, a rod lens fused to an end of the optical fiber, and a prism having a polygonal section fused to a distal end surface of the rod lens.

The optical fiber and the rod lens, and the rod lens and the prism are joined to each other by fusing. Thus, there is no adhesive layer in an optical path, and accordingly, there are no variations in beam quality, no degradation in beam quality due to detachment of the adhesion, and no complete detachment of the prism. Production can be easily performed using related-art known fiber welding equipment.
Since the lateral light emitting device needs no shaft or sheath, the outer diameter can be made to be very small. Thus, the lateral light emitting device can be used for a very thin blood vessel and the like.

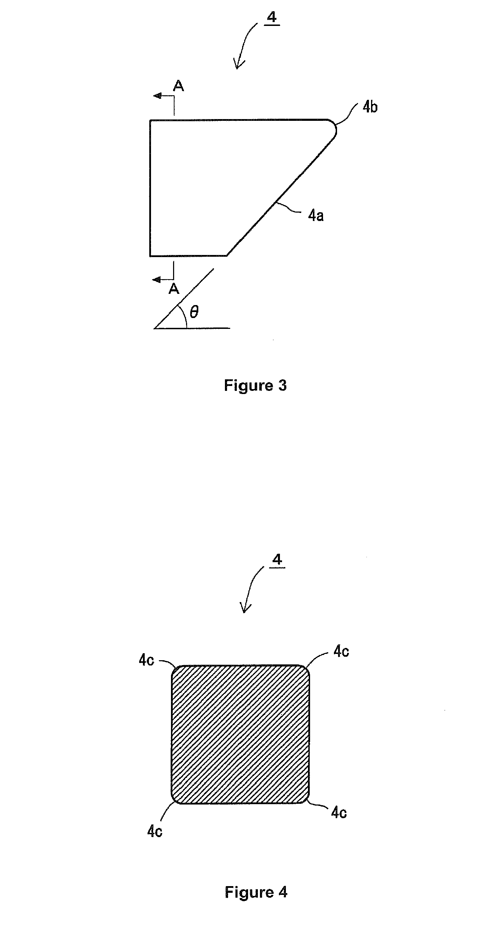

In most cases, the optical fiber is a single-mode optical fiber. However, the optical fiber may use a polarization maintaining fiber or a multi-mode fiber. In addition, the optical fiber may even use a bundle fiber for image transmission.
The rod lens needs to be formed of silica-based glass in order to undergo fusing. The rod lens may use a so-called GI-type fiber, of which the core has a refractive index distribution, or a so-called GRIN lens, of which the entire section has a refractive index distribution.
The rod lens may also use a lens produced by fusing together two types of (or three or more types of) GRIN lenses each having a different numerical aperture as described in Japanese Unexamined Patent Application Publication No. 2005-115097.
The prism needs to be formed of silica-based glass-based material in order to undergo fusing. A typical inclination angle (θ in Fig. 3) of the distal end inclined surface of the prism relative to the axis is 45°. In this case, light is laterally emitted at 90° relative to the axis. By changing the inclination angle (θ) of the distal end inclined surface, the light emission angle can be changed. Coating such as mirror-coating (coated with Au or the like) or half-mirror coating (coated with a dielectric multi-layer or the like) may be applied to the distal end inclined surface according to need.
In the prism having a polygonal section according to the present invention, the section taken in a direction perpendicular to the incident optical axis has a triangular, quadrangular, or another polygonal shape.

### [Claim 2]

Also according to the present invention, in the lateral light emitting device according to Claim 1, a distal end acute angle portion of the prism is chamfered.

By chamfering the distal end acute angle portion of the prism, when the lateral light emitting device is directly inserted into the subject body without being covered with a sheath, it is less likely that the subject body is damaged.
Chamfering includes a method, for example, using electric discharge machining or laser machining in order to smooth the distal end acute angle portion of the prism into a curved surface-like shape.

### [Claim 3]

Also according to the present invention, in the lateral light emitting device according to Claim 1 or 2, an outer diameter of the rod lens is in a range from 124 to 250 µm.

When the outer diameters of the optical fiber and the rod lens are close to each other, the axes thereof are automatically aligned with each other by self-alignment effects due to surface tension caused in fusing. This significantly reduces coupling loss between the optical fiber and the rod lens. When the outer diameter of the optical fiber is 125 µm, the outer diameter of the rod lens is suitably from 124 to 250 µm. This also meets the purpose of the device that is intended to be inserted into a thin blood vessel and the like.

### [Claim 4]

Also according to the present invention, in the lateral light emitting device according to any one of Claims 1 to 3, a maximum diameter of the prism is equal to or smaller than an outer diameter of the rod lens.

With the maximum diameter (for example, the length of the diagonal line of a square shape) of the prism set to a length equal to or smaller than the outer diameter of the rod lens, corner portions of the polygonal section of the prism do not protrude outward beyond an outer peripheral surface of the rod lens. This can prevent the subject body from being damaged by the corner portions of the prism.

### [Claim 5]

Also according to the present invention, in the lateral light emitting device according to any one of Claims 1 to 4, the prism has a quadrangular section having chamfered corner portions.

When the corner portions of the quadrangular section of the prism are chamfered, the subject body can be prevented from being damaged by the corner portions of the prism.

### [Claim 6]

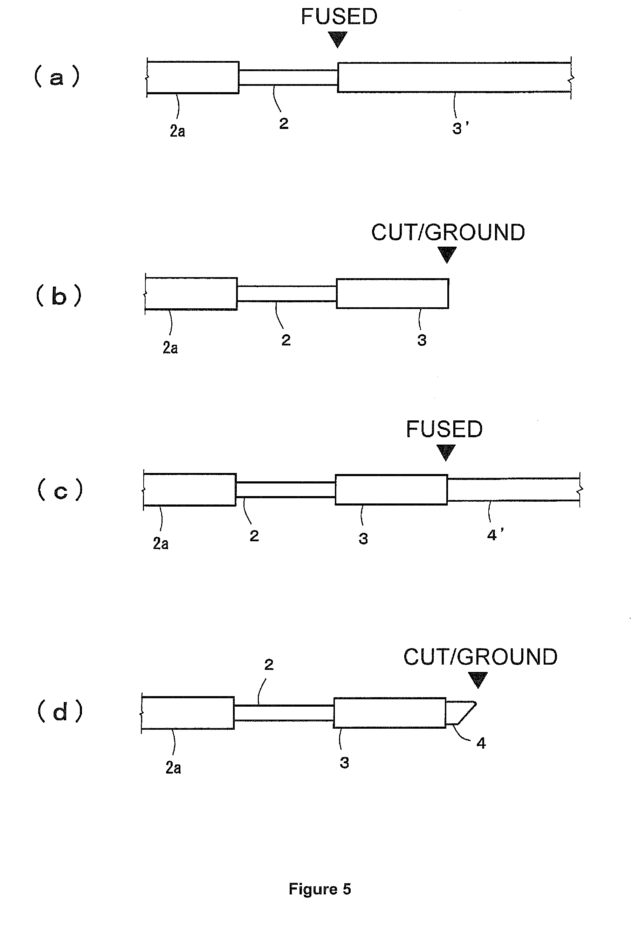

Also according to the present invention, a method of producing a lateral light emitting device includes a step of fusing a lens fiber to one end of an optical fiber, a step of cutting the lens fiber to a specified length so as to form a rod lens, a step of fusing a polygonal fiber to an end surface of the rod lens, and a step of cutting and grinding the polygonal fiber so as to form a distal end inclined surface to produce a prism (FIG. 5).

### [Claim 7]

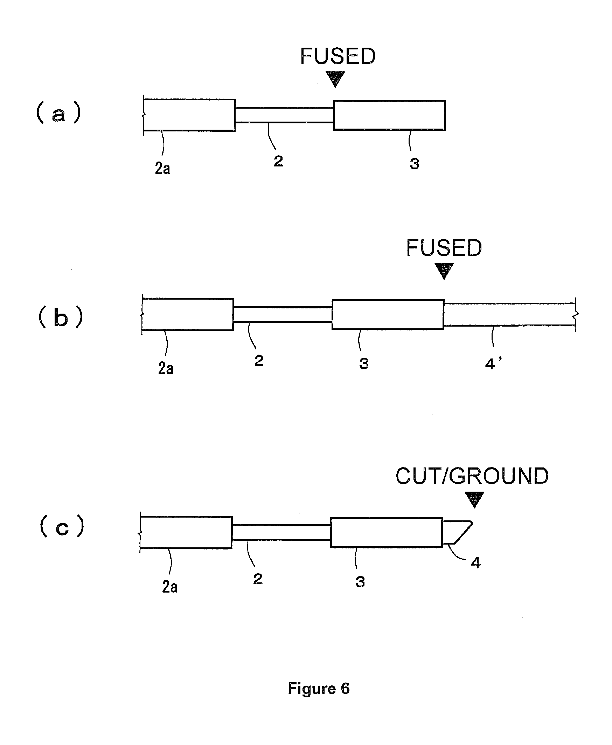

Also according to the present invention, a method of producing a lateral light emitting device includes a step of fusing a rod lens to one end of an optical fiber, a step of fusing a polygonal fiber to an end surface of the rod lens, and a step of cutting and grinding the polygonal fiber so as to form a distal end inclined surface to produce a prism (Fig. 6).

### [Claim 8]

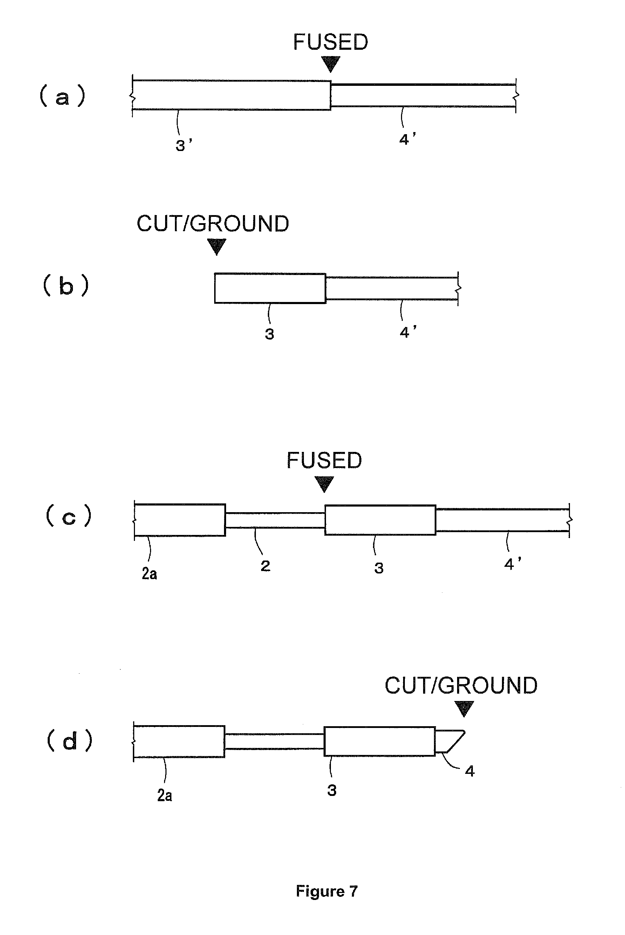

Also according to the present invention, a method of producing a lateral light emitting device includes a step of fusing a polygonal fiber to one end of a lens fiber, a step of cutting the lens fiber to a specified length so as to form a rod lens, a step of fusing an end surface of the rod lens to one end of an optical fiber, and a step of cutting and grinding the polygonal fiber so as to form a distal end inclined surface to produce a prism (Fig. 7).

### [Claim 9]

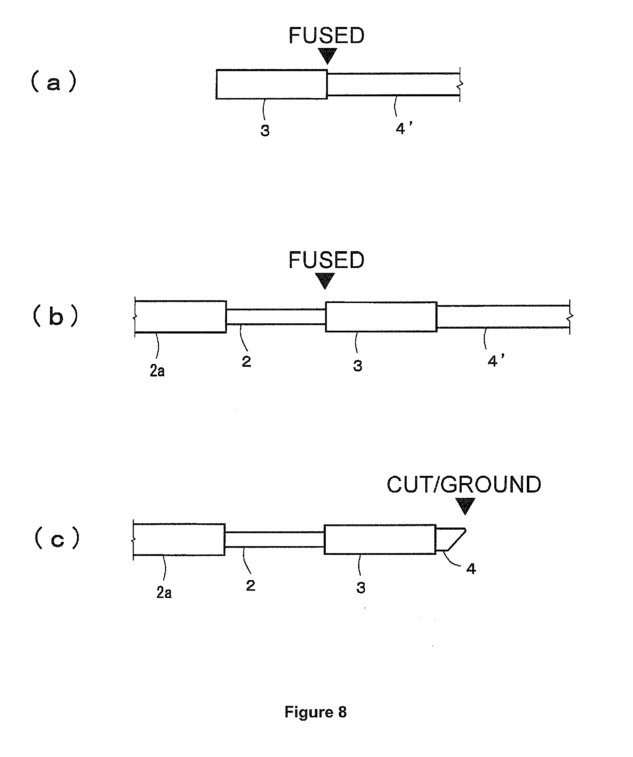

Also according to the present invention, a method of producing a lateral light emitting device includes a step of fusing a polygonal fiber to one end of a rod lens, a step of fusing an end surface of the rod lens to one end of an optical fiber, and a step of cutting and grinding the polygonal fiber so as to form a distal end inclined surface to produce a prism (Fig. 8).

### [Claim 10]

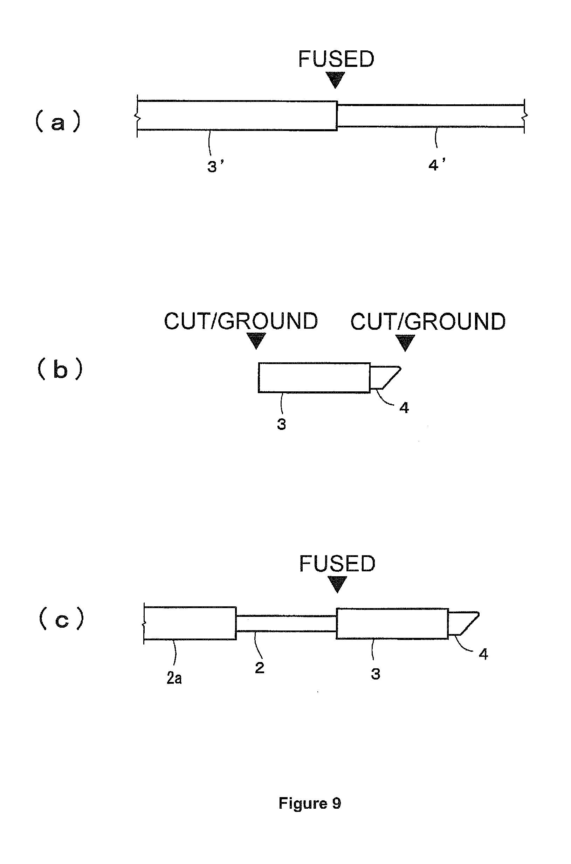

Also according to the present invention, a method of producing a lateral light emitting device includes a step of fusing a polygonal fiber to one end of a lens fiber, a step of cutting the lens fiber to a specified length so as to form a rod lens and cutting and grinding the polygonal fiber so as to form a distal end inclined surface to produce a prism, thereby producing a rod lens with a prism, and a step of fusing the rod lens with a prism to one end of an optical fiber (Fig. 9).

### [Claim 11]

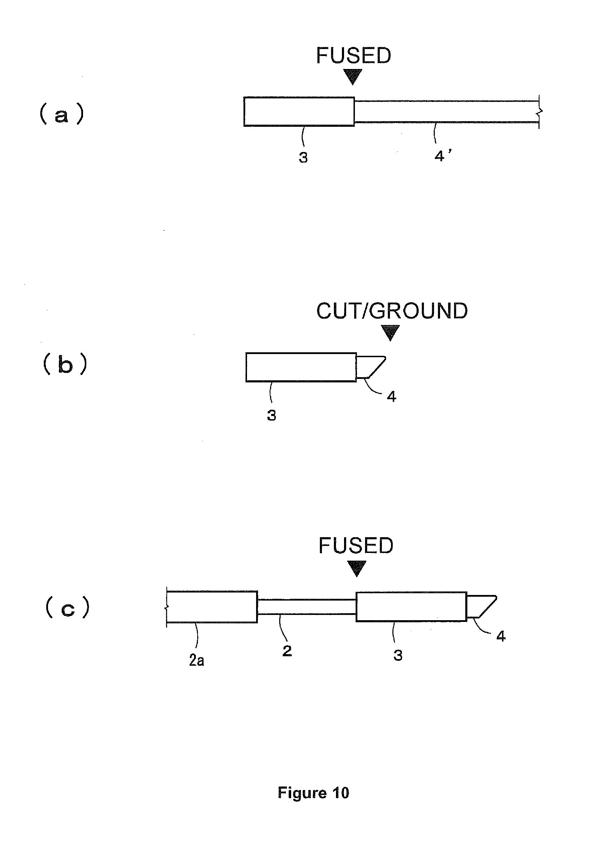

Also according to the present invention, a method of producing a lateral light emitting device includes a step of fusing a polygonal fiber to one end of a rod lens, a step of cutting and grinding the polygonal fiber so as to form a distal end inclined surface to produce a prism, thereby producing a rod lens with a prism, and a step of fusing the rod lens with a prism to one end of an optical fiber (Fig. 10).

Using any one of these production methods, the lateral light emitting device according to claim 1 can be easily produced.
Use of the lens fiber, which has not been cut so as to form a rod lens, and the polygonal fiber, which has not been cut so as to form a prism, for fusing facilitates handling in a fusing process. This can facilitate production of the lateral light emitting device according to the present invention.
When the rod lens has a length at which the rod lens is suitably fused, not only the lens fiber but also the rod lens may be directly fused.

### [Claim 12]

Also according to the present invention, in the method of producing the lateral light emitting device according to any one of Claims 6 to 11, the step of cutting and grinding the polygonal fiber so as to form the distal end inclined surface to produce the prism includes chamfering of a distal end acute angle portion of the prism.

The present invention includes the method of producing the lateral light emitting device according to Claim 2.
Chamfering may be performed, for example, using electric discharge machining. When electric current is discharged to the distal end acute angle portion of the prism, the distal end portion is melted and then solidified so as to form a curved surface-like shape due to surface tension of molten glass.

### [Claim 13]

Also according to the present invention, in the method of producing the lateral light emitting device according to any one of Claims 6 to 12, an outer diameter of the rod lens is in a range from 124 to 250 µm.

The present invention includes the method of producing the lateral light emitting device according to Claim 3.

### [Claim 14]

Also according to the present invention, in the method of producing the lateral light emitting device according to any one of Claims 6 to 13, a maximum diameter of the prism is equal to or smaller than an outer diameter of the rod lens.

The present invention includes the method of producing the lateral light emitting device according to Claim 4.

### [Claim 15]

Also in the present invention, in the method of producing the lateral light emitting device according to any one of Claims 6 to 15, the polygonal fiber is produced by drawing a prism-shaped silica-based glass base material.

When the prism-shaped silica-based glass base material is drawn, the glass is softened and the corner portions of the section of the polygonal fiber are rounded. Thus, the lateral light emitting device according to Claim 5 can be easily produced.

### Advantageous Effects of Invention

In the lateral light emitting device according to the present invention, no adhesive is used. Thus, variations in beam quality due to an adhesive layer are eliminated.
Since the optical fiber and the rod lens, and the rod lens and the prism are joined to each other by welding in an integral manner, there is no possibility of detachment of the adhesion in the joined portions, the detachment being detachment that may degrade beam quality, or no possibility of complete detachment of the prism or the rod lens that may leave the prism or the rod lens in the subject body. Thus, the lateral light emitting device needs not be covered with the sheath.
Since the lateral light emitting device needs no shaft or sheath as required for the related art, the outer diameter of the lateral light emitting device can be made to be very small. Thus, the lateral light emitting device can be inserted into and used for a very thin blood vessel and the like.
With any one of the methods of producing the lateral light emitting device according to the present invention, the lateral light emitting device according to the present invention can be easily produced.

### Brief Description of Drawings

[Fig. 1] Fig. 1 is a side view of a lateral light emitting device 1 according to an embodiment.
[Fig. 2] Fig. 2 is a front view of the lateral light emitting device 1.
[Fig. 3] Fig. 3 is an enlarged side view of a prism 4.
[Fig. 4] Fig. 4 is a sectional view of the prism 4 illustrated in Fig. 3 taken along line A-A.
[Fig. 5] Fig. 5 is an explanatory view illustrating a production method of the lateral light emitting device according to the embodiment.
[Fig. 6] Fig. 6 is an explanatory view illustrating a production method of the lateral light emitting device according to the embodiment.
[Fig. 7] Fig. 7 is an explanatory view illustrating a production method of the lateral light emitting device according to the embodiment.
[Fig. 8] Fig. 8 is an explanatory view illustrating a production method of the lateral light emitting device according to the embodiment.
[Fig. 9] Fig. 9 is an explanatory view illustrating a production method of the lateral light emitting device according to the embodiment.
[Fig. 10] Fig. 10 is an explanatory view illustrating a production method of the lateral light emitting device according to the embodiment.
[Fig. 11] Fig. 11 is a side view of a lateral light emitting device 1' according to an embodiment.
[Fig. 12] Fig. 12 is a sectional explanatory view of a related-art lateral light emitting device.

### Description of Embodiments

### Embodiment

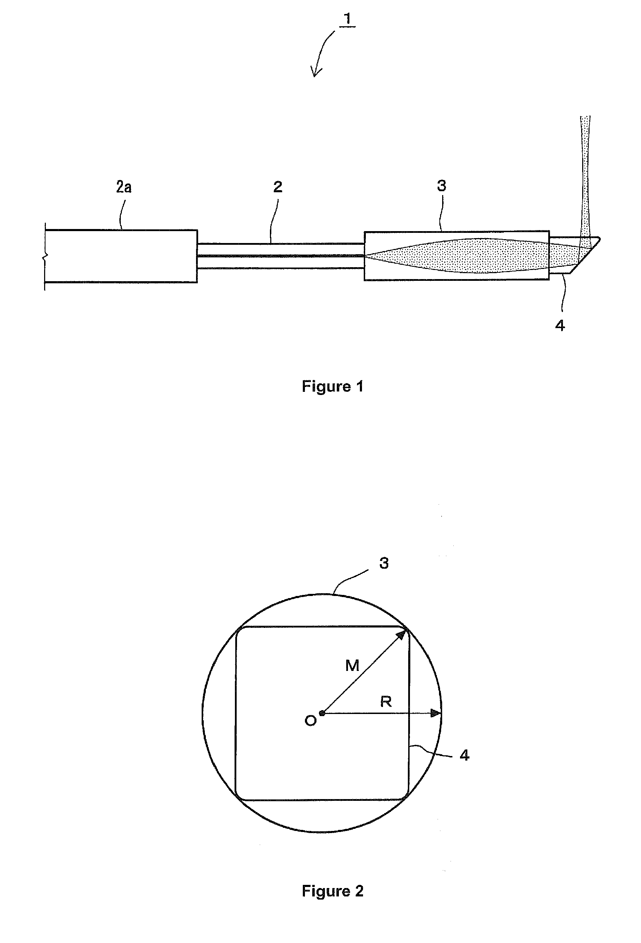

Figs. 1 to 4 relate to a lateral light emitting device 1 of an embodiment. Fig. 1 is a side view, Fig. 2 is a front view, Fig. 3 is an enlarged side view of a prism 4, and Fig. 4 is a sectional view of the prism 4 illustrated in Fig. 3 taken along line A-A.
The lateral light emitting device 1 includes an optical fiber 2, a rod lens 3, and the prism 4.
The optical fiber 2 is a single-mode optical fiber having an outer diameter of 125 µm. A coating 2a is removed from the distal end of the optical fiber 2, and the rod lens 3 is fused to the distal end surface of the optical fiber 2.
The rod lens 3 is a GRIN lens formed of silica-based glass and has an outer diameter of 250 µm. When the optical fiber 2 and the rod lens 3 are fused to each other, the axes thereof are automatically aligned with each other by self-alignment effects.
The prism 4 is formed of silica glass, the section of which has a square shape defined by sides each having a length of about 175 µm. The prism has a distal end inclined surface 4a formed by diagonally grinding a distal end portion thereof. The prism has a distal end acute angle portion 4b at the most distal end thereof. The distal end acute angle portion 4b is chamfered so as to have a curved surface (Fig. 3).
The maximum diameter M of the prism 4 is equal to an outer diameter R (250 µm) of the rod lens 3, and the shape of the prism 4 is inscribed in that of the rod lens 3 (Fig. 2).
Corner portions 4c of the section of the prism 4 (other than the section of the distal end inclined surface 4a) perpendicular to the axis of the prism 4 are chamfered so as to have curved shapes (Fig. 4).
The prism 4 is produced as follows. That is, a prism-shaped silica-based glass base material is drawn into a polygonal fiber, which is cut and diagonally ground so as to form the distal end inclined surface 4a. After that, the distal end acute angle portion 4b is chamfered using electric discharge machining and the distal end inclined surface 4a is Au-coated.

The lateral light emitting device 1 can be easily produced using any one of methods illustrated in Figs. 5 to 10.
Fig. 5 illustrates a case of production described in Claim 6, including a step of fusing a lens fiber to one end of an optical fiber, a step of cutting the lens fiber to a specified length so as to form a rod lens, a step of fusing a polygonal fiber to an end surface of the rod lens, and a step of cutting and grinding the polygonal fiber so as to form a distal end inclined surface to produce a prism.
In the drawing, (a) illustrates a state in which a lens fiber 3' is fused to one end of the optical fiber 2, (b) illustrates a state in which the lens fiber 3' is cut to a specified length (after the cutting, the cut surface may be ground according to need) so as to produce the rod lens 3, (c) illustrates a state in which a polygonal fiber 4' is fused to an end surface of the rod lens 3, and (d) illustrates a state in which the polygonal fiber 4' is cut and ground so as to form the distal end inclined surface, and then the distal end acute angle portion 4b is chamfered and the distal end inclined surface 4a is Au-coated so as to produce the prism 4.

Fig. 6 illustrates a case of production described in Claim 7, including a step of fusing a rod lens to one end of an optical fiber, a step of fusing a polygonal fiber to an end surface of the rod lens, and a step of cutting and grinding the polygonal fiber so as to form a distal end inclined surface to produce a prism.
In the drawing, (a) illustrates a state in which the rod lens 3 is fused to the one end of the optical fiber 2, (b) illustrates a state in which the polygonal fiber 4' is fused to the end surface of the rod lens 3, and (c) illustrates a state in which the polygonal fiber 4' is cut and ground so as to form the distal end inclined surface, and then the distal end acute angle portion 4b is chamfered and the distal end inclined surface 4a is Au-coated so as to produce the prism 4.

Fig. 7 illustrates a case of production described in Claim 8, including a step of fusing a polygonal fiber to one end of a lens fiber, a step of cutting the lens fiber to a specified length so as to form a rod lens, a step of fusing an end surface of the rod lens to one end of an optical fiber, and a step of cutting and grinding the polygonal fiber so as to form a distal end inclined surface to produce a prism.
In the drawing, (a) illustrates a state in which the polygonal fiber 4' is fused to the one end of the lens fiber 3', (b) illustrates a state in which the lens fiber 3' is cut to a specified length (after the cutting, the cut surface may be ground according to need) so as to produce the rod lens 3, (c) illustrates a state in which the end surface of the rod lens 3 is fused to the one end of the optical fiber 2, and (d) illustrates a state in which the polygonal fiber 4' is cut and ground so as to form the distal end inclined surface, and then the distal end acute angle portion 4b is chamfered and the distal end inclined surface 4a is Au-coated so as to produce the prism 4.

Fig. 8 illustrates a case of production described in Claim 9, including a step of fusing a polygonal fiber to one end of a rod lens, a step of fusing an end surface of the rod lens to one end of an optical fiber, and a step of cutting and grinding the polygonal fiber so as to form a distal end inclined surface to produce a prism.
In the drawing, (a) illustrates a state in which the polygonal fiber 4' is fused to one end of the rod lens 3, (b) illustrates a state in which the end surface of the rod lens 3 is fused to the one end of the optical fiber 2, and (c) illustrates a state in which the polygonal fiber 4' is cut and ground so as to form the distal end inclined surface, and then the distal end acute angle portion 4b is chamfered and the distal end inclined surface 4a is Au-coated so as to produce the prism 4.

Fig. 9 illustrates a case of production described in Claim 10, including a step of fusing a polygonal fiber to one end of a lens fiber, a step of cutting the lens fiber to a specified length so as to form a rod lens and cutting and grinding the polygonal fiber so as to form a distal end inclined surface to produce a prism, thereby producing a rod lens with a prism, and a step of fusing the rod lens with a prism to one end of an optical fiber.
In the drawing, (a) illustrates a state in which the polygonal fiber 4' is fused to the one end of the lens fiber 3', (b) illustrates a state in which the lens fiber 3' is cut to a specified length (after the cutting, the cut surface may be ground according to need) so as to produce the rod lens 3, and the polygonal fiber is cut and ground so as to form the distal end inclined surface, and then the distal end acute angle portion 4b is chamfered and the distal end inclined surface 4a is Au-coated so as to produce the prism 4, thereby producing a rod lens with a prism, and (c) illustrates a state in which the rod lens with a prism is fused to the one end of the optical fiber 2.

Fig. 10 illustrates a case of production described in Claim 11, including a step of fusing a polygonal fiber to one end of a rod lens, a step of cutting and grinding the polygonal fiber so as to form a distal end inclined surface to produce a prism, thereby producing a rod lens with a prism, and a step of fusing the rod lens with a prism to one end of an optical fiber.
In the drawing, (a) illustrates a state in which the polygonal fiber 4' is fused to the one end of the rod lens 3, (b) illustrates a state in which the polygonal fiber is cut and ground so as to form the distal end inclined surface, and then the distal end acute angle portion 4b is chamfered and the distal end inclined surface 4a is Au-coated so as to produce the prism 4, thereby producing the rod lens with a prism, and (c) illustrates a state in which the rod lens with a prism is fused to the one end of the optical fiber 2.

In a lateral light emitting device 1' illustrated in Fig. 11, the rod lens 3 has an outer diameter of 125 µm, which is equal to the outer diameter of the optical fiber 2. The prism 4 has a size at which the shape of the prism 4 is inscribed in that of the rod lens 3. Other structures and the methods of producing the lateral light emitting device 1' are completely the same as those of the lateral light emitting device 1.
The lateral light emitting device 1' has a diameter further smaller than that of the lateral light emitting device 1 and is suitably used for very thin blood vessels and the like.

### Industrial Applicability

The lateral light emitting device according to the present invention can be used as an optical probe for OCT, and in addition, used as a fiber-optic module for optical communication, for example, for combining a laser diode and a single-mode fiber, an optical probe for distance and displacement sensors, an optical probe for an endoscope, and so forth.

According to the present invention, the inclined surface can be also formed at the distal end of the prism by cutting the polygonal fiber using a laser so as to form the inclined surface other than cutting and grinding.

### Reference Signs List

- 1: lateral light emitting device
- 2: optical fiber
- 2a: coating
- 3: rod lens
- 3': lens fiber
- 4: prism
- 4a: distal end inclined surface
- 4b: distal end acute angle portion
- 4c: angle portion
- 4': polygonal fiber
- 5: shaft
- 6: distal end holding portion
- 7: sheath

## Claims

1. A lateral light emitting device comprising an optical fiber, a rod lens fused to an end of the optical fiber, and a prism having a polygonal section fused to a distal end surface of the rod lens.

2. The lateral light emitting device according to Claim 1, wherein a distal end acute angle portion of the prism is chamfered.

3. The lateral light emitting device according to Claim 1 or 2, wherein an outer diameter of the rod lens is in a range from 124 to 250 µm.

4. The lateral light emitting device according to any one of Claims 1 to 3, wherein a maximum diameter of the prism is equal to or smaller than an outer diameter of the rod lens.

5. The lateral light emitting device according to any one of Claims 1 to 4, wherein the prism has a quadrangular section, the quadrangular section having chamfered corner portions.

6. A method of producing a lateral light emitting device comprising a step of fusing a lens fiber to one end of an optical fiber, a step of cutting the lens fiber to a specified length so as to form a rod lens, a step of fusing a polygonal fiber to an end surface of the rod lens, and a step of cutting and grinding the polygonal fiber so as to form a distal end inclined surface to produce a prism.

7. A method of producing a lateral light emitting device comprising a step of fusing a rod lens to one end of an optical fiber, a step of fusing a polygonal fiber to an end surface of the rod lens, and a step of cutting and grinding the polygonal fiber so as to form a distal end inclined surface to produce a prism.

8. A method of producing a lateral light emitting device comprising a step of fusing a polygonal fiber to one end of a lens fiber, a step of cutting the lens fiber to a specified length so as to form a rod lens, a step of fusing an end surface of the rod lens to one end of an optical fiber, and a step of cutting and grinding the polygonal fiber so as to form a distal end inclined surface to produce a prism.

9. A method of producing a lateral light emitting device comprising a step of fusing a polygonal fiber to one end of a rod lens, a step of fusing an end surface of the rod lens to one end of an optical fiber, and a step of cutting and grinding the polygonal fiber so as to form a distal end inclined surface to produce a prism.

10. A method of producing a lateral light emitting device comprising a step of fusing a polygonal fiber to one end of a lens fiber, a step of cutting the lens fiber to a specified length so as to form a rod lens and cutting and grinding the polygonal fiber so as to form a distal end inclined surface to produce a prism, thereby producing a rod lens with a prism, and a step of fusing the rod lens with a prism to one end of an optical fiber.

11. A method of producing a lateral light emitting device comprising a step of fusing a polygonal fiber to one end of a rod lens, a step of cutting and grinding the polygonal fiber so as to form a distal end inclined surface to produce a prism, thereby producing a rod lens with a prism, and a step of fusing the rod lens with a prism to one end of an optical fiber.

12. The method of producing the lateral light emitting device according to any one of Claims 6 to 11, wherein the step of cutting and grinding the polygonal fiber so as to form the distal end inclined surface to produce the prism includes chamfering of a distal end acute angle portion of the prism.

13. The method of producing the lateral light emitting device according to any one of Claims 6 to 12, wherein an outer diameter of the rod lens is in a range from 124 to 250 µm.

14. The method of producing the lateral light emitting device according to any one of Claims 6 to 13, wherein a maximum diameter of the prism is equal to or smaller than an outer diameter of the rod lens.

15. The method of producing the lateral light emitting device according to any one of Claims 6 to 15, wherein the polygonal fiber is produced by drawing a prism-shaped silica-based glass base material.
